# EUROPEAN PATENT SPECIFICATION

(11) **EP 3 445 453 B1**
(45) Date of publication and mention of the grant of the patent: **25.06.2025**
(21) Application number: 17712143.1
(22) Date of filing: 21.03.2017
(51) Int. Cl.: A61Q 11/00, A61K 8/30

(54) **APPARATUS FOR DEMONSTRATING THE EFFECT OF BLUE LIGHT ON TEETH**
APPARAT ZUR DEMONSTRATION DER WIRKUNG VON BLAUEM LICHT AUF DIE ZÄHNE
APPAREIL POUR DÉMONTRER L'EFFET DE LA LUMIÈRE BLEUE SUR LES DENTS

(30) Priority: 21.04.2016 WO PCT/EP2016/166373
(43) Date of publication of application: 27.02.2019
(73) Proprietor: Unilever IP Holdings B.V., 3013 AL Rotterdam (NL); Unilever Global IP Limited, Wirral, Merseyside CH62 4ZD (GB)
(72) Inventor: ASHCROFT, Alexander, Thomas, Mollington Cheshire, CH1 6LD (GB); JOINER, Andrew, Wirral Merseyside CH63 3JW (GB); KNOTT, Daniel, James, Wirral Merseyside CH63 3JW (GB); LIMER, Adam, John, Wirral Merseyside CH63 3JW (GB); LITTLEWOOD, David, Thomas, Wirral Merseyside CH63 3JW (GB); PHILPOTTS, Carole, Jane, Wirral Merseyside CH63 3JW (GB)
(74) Representative: Tansley, Sally Elizabeth
(86) International application number: PCT/EP2017/056709
(87) International publication number: WO 2017/182219

(56) References cited:
- EP-A1- 1 935 395
- US-A- 6 030 222
- PRIDMORE RALPH W.: "Complementary colors: The structure of wavelength discrimination, uniform hue, spectral sensitivity, saturation, chromatic adaptation, and chromatic induction", COLOR RESEARCH & APPLICATION, vol. 34, no. 3, 1 June 2009 (2009-06-01), US, pages 233 - 252, XP055826315, ISSN: 0361-2317, DOI: 10.1002/col.20490
- GLOBALSYSTEMSSCIENCE: "Mixing yellow and blue light", 11 April 2015 (2015-04-11), pages 1, XP054982079, Retrieved from the Internet <URL:https://www.youtube.com/watch?v=ef_MRXlmVt0> [retrieved on 20210722]
- ANDREW JOINER: "A silica toothpaste containing blue covarine: a new technological breakthrough in whitening", INTERNATIONAL DENTAL JOURNAL, 1 October 2009 (2009-10-01), pages 284 - 288, XP055292722, Retrieved from the Internet <URL:http://onlinelibrary.wiley.com/store/10.1922/IDJ_2261Joiner05/asset/IDJ_2261Joiner05.pdf?v=1&t=ird6y4i7&s=724515f66226a601b3c195992f557502c42add16> [retrieved on 20160802], DOI: 10.1922/IDJ_2261Joiner05
- DATABASE GNPD [online] MINTEL; October 2012 (2012-10-01), ANONYMOUS: "ReelRedSweet2010", XP002760668, Database accession no. 1909862

## Description

The present invention relates to an apparatus for demonstrating the effect that a source of blue light has on the teeth.

The addition of blue pigment has been associated with tooth whitening as described in EP 1 935 395. However, consumers have difficulty in associating the addition of a blue with a corresponding diminishing of yellow coloration. There remains a need for a simple demonstration to show that the addition of blue can cause yellowing of teeth to disappear without the teeth appearing blue.

Accordingly the present invention relates to an apparatus or demonstrating the effect that blue light has on the teeth comprising:
i) a transparent/translucent container comprising titanium dioxide and a yellow colorant with a wavelength 570nm to 590nm;
ii) a light source having a wavelength from 450 nm to 495 nm;
iii) means of shining the light source onto the solution or gel.

Particularly beneficial results are achieved in diminishing the yellow appearance of the yellow solution when the light source is blue. The more intense the yellow of the solution the stronger the blue light needs to be to counter the effect.

Preferably, the colorant of the solution is a dye, preferably a water soluble dye, more preferably it is a yellow food paste. It is preferred if the colorant is mixed with water. The solution or gel comprises titanium dioxide. Titanium dioxide can enhance the whitening appearance given by blue light. Preferably the level of titanium dioxide in the solution is from 0.0001 to 0.001 % w/w of the total solution.

The light source of step may be immersed in the solution or gel or may be external to the solution or gel.

Any light source having the required wavelength is suitable for use in the invention however LED light sources are particularly preferred.

It is preferable if a solution is used rather than a gel.

The container for the solution of gel should be transparent or translucent. Clear, uncoloured glass is particularly effective.

The method of use of the apparatus can result in the visualisation of the solution/gel occurring sequentially (in that the same solution/gel is used for the visualisation steps ii) and iv)) or simultaneously (in that different solution/gels having identical compositions are used for the visualisation steps ii) and iv)).

### EXAMPLE 1 (not according to the invention)

a) 0.1g Yellow Food paste was added to 100g of Demin/Milli Q water. The solution was shaken well until dye is fully dispersed to prepare a stock solution. The stock solution was diluted by adding 1.0g solution to 100g water and shaken to mix. Identical amounts of the solution were placed in identical glass jars. A LED blue light was shone on one jar and the two jars visually compared.

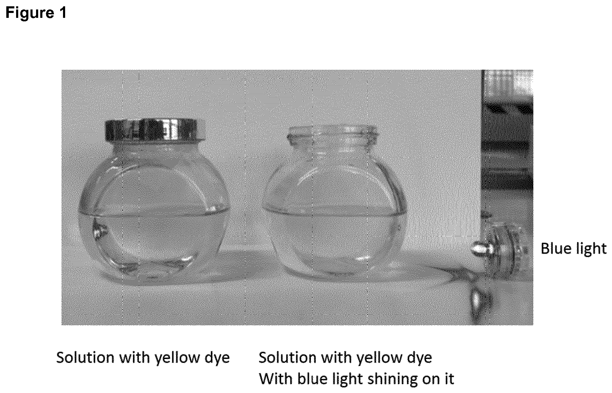

Fig 1 is a photograph of the results showing two jars containing identical solutions when having blue light shone on it and one without blue light.
It can be seen that the solution of the jar which has the blue light shone on it has lost its yellow appearance but does not appear blue.

### EXAMPLE 2

a) 0.1g Yellow Food paste was added to 100g of Demin/Milli Q water. The solution was shaken well until dye is fully dispersed to prepare a stock solution. The stock solution was diluted by adding 1.5g solution to 100g water and shaken to mix to give Solution A.
b) 1.0g of Titanium Dioxide was added to another 100g of Demin/Milli Q water. The mixture was shaken well and 0.02-0.03g of this mixture was added to 100g of solution A. The container was visually compared with and without a LED blue light being shone on the container.

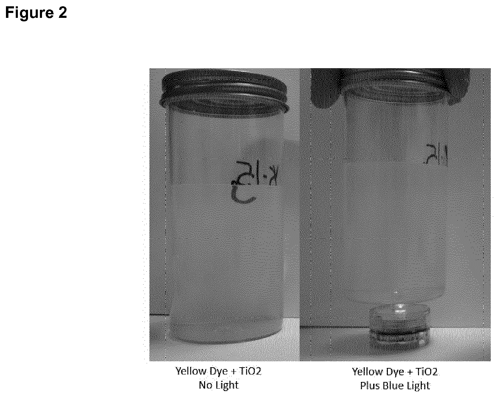

Fig 2 is a photograph of the results showing the same container containing the mixture of the yellow dye and titanium dioxide with and without having a blue light shone on it.

It can be seen that the mixture of the container has lost its yellow appearance when the blue light is shone on it but does not appear blue.

## Claims

1. Apparatus for demonstrating the whitening effect blue light has on the teeth comprising:
i) a transparent/translucent container containing a solution or gel comprising titanium dioxide and a yellow colorant with a wavelength 570nm to 590nm;
ii) a light source having a wavelength from 450 nm to 495 nm;
iii) means of shining the light source onto the solution or gel.

2. Apparatus according to claim 1 in which the light source is blue.

3. Apparatus according to any preceding claim in which the colourant is a dye.

4. Apparatus according to any preceding claim in which the colourant is Quinoline Yellow (E104).

5. Apparatus according to any preceding claimin which the level of titanium dioxide is from 0.0001 to 0.001 % w/w of the total solution.

6. Apparatus according to any preceding claim in which the light source of point ii) is immersed in the solution or gel.

7. Apparatus according to any of claims 1 to 5 in which the light source of point ii) is external to the solution or gel.

## Patentansprüche

1. Vorrichtung zur Demonstration der aufhellenden Wirkung von blauem Licht auf die Zähne, die Folgendes umfasst:
i) einen transparenten bzw. transluzenten Behälter, der eine Lösung oder ein Gel beinhaltet, das Titandioxid und ein gelbes Färbemittel mit einer Wellenlänge von 570 nm bis 590 nm enthält;
ii) eine Lichtquelle mit einer Wellenlänge von 450 nm bis 495 nm;
iii) Mittel zum Bestrahlen der Lösung oder des Gels mit der Lichtquelle.

2. Vorrichtung nach Anspruch 1, in der die Lichtquelle blau ist.

3. Vorrichtung nach einem vorhergehenden Anspruch, in der das Färbemittel ein Farbstoff ist.

4. Vorrichtung nach einem vorhergehenden Anspruch, in der das Färbemittel Chinolin-Gelb (E104) ist.

5. Vorrichtung nach einem vorhergehenden Anspruch, in dem der Gehalt an Titandioxid von 0,0001 bis 0,001 %w/w der Gesamtlösung beträgt.

6. Vorrichtung nach einem vorhergehenden Anspruch, in der die Lichtquelle von Punkt ii) in der Lösung oder dem Gel eingetaucht ist.

7. Vorrichtung nach einem der Ansprüche 1 bis 5, in der sich die Lichtquelle von Punkt ii) außerhalb der Lösung oder des Gels befindet.

## Revendications

1. Appareil pour démontrer l'effet blanchissant qu'une lumière bleue a sur les dents, comprenant :
i) un récipient transparent/translucide contenant une solution ou un gel comprenant du dioxyde de titane et un colorant jaune ayant une longueur d'onde de 570 nm à 590 nm ;
ii) une source de lumière ayant une longueur d'onde de 450 nm à 495 nm ;
iii) des moyens pour illuminer la solution ou le gel avec la source de lumière.

2. Appareil selon la revendication 1, dans lequel la source de lumière est bleue.

3. Appareil selon l'une quelconque des revendications précédentes, dans lequel le colorant est une teinture.

4. Appareil selon l'une quelconque des revendications précédentes, dans lequel le colorant est le jaune de quinoléine (E104).

5. Appareil selon l'une quelconque des revendications précédentes, dans lequel le taux de dioxyde de titane est de 0,0001 à 0,001 % en poids/poids de la solution totale.

6. Appareil selon l'une quelconque des revendications précédentes, dans lequel la source de lumière du point ii) est immergée dans la solution ou le gel.

7. Appareil selon l'une quelconque des revendications 1 à 5, dans lequel la source de lumière du point ii) est externe à la solution ou au gel.
